# EUROPEAN PATENT SPECIFICATION

(11) **EP 1 332 717 B1**
(45) Date of publication and mention of the grant of the patent: **18.08.2004**
(21) Application number: 03000087.1
(22) Date of filing: 08.01.2003
(51) Int. Cl.: A61B 5/022

(54) **Cuff for blood pressure monitor**
Manschette für ein Blutdruckmessgerät
Manchette pour un sphygmomanomètre

(30) Priority: 04.02.2002 JP 2002026424
(43) Date of publication of application: 06.08.2003
(73) Proprietor: Omron Healthcare Co., Ltd., Kyoto 600-0084 (JP)
(72) Inventor: Inagaki, Takashi, Omron Corp, 801 Minamifudodo-cho, Kyoto-shi, Kyoto 600-8530 (JP); Sano, Yoshihiko, Omron Corp., 801 Minamifudodo-cho, Kyoto-shi, Kyoto 600-8530 (JP); Taniguchi, Minoru, Omron Corp 801 Minamifudodo-cho, Kyoto-shi, Kyoto 600-8530 (JP)
(74) Representative: Kilian, Helmut, Dr.

(56) References cited:
- JP-A- 61 238 229
- US-A- 4 572 205
- PATENT ABSTRACTS OF JAPAN vol. 018, no. 257 (C-1200), 17 May 1994 (1994-05-17) & JP 06 038931 A (MATSUSHITA ELECTRIC WORKS LTD;OTHERS: 01), 15 February 1994 (1994-02-15)

## Description

### BACKGROUND OF THE INVENTION

### Field of the Invention

The present invention relates to a cuff for a blood pressure monitor and, more particularly, to a cuff characterized by a curled elastic member disposed on the outside of a bladder to hold a ring form of the cuff.

### Description of the Related Art

As shown in Fig. 6 (perspective view) and Fig. 7 (section view), a cuff for a blood pressure monitor has generally a configuration such that a bladder 31 and a curled elastic member 32 are provided in a cloth bag 30. A tube 33 is connected to the bladder 31, and a Velcro fastener 34 is attached on the outside of the cloth bag 30. The curled elastic member 32 is disposed on the outside of the bladder 31 to hold the cuff in a ring form by its elasticity. As shown in Fig. 8, the curled elastic member 32 has a uniform thickness and has a sectional shape of a complete round in which a part thereof is a discontinuous portion 32a.

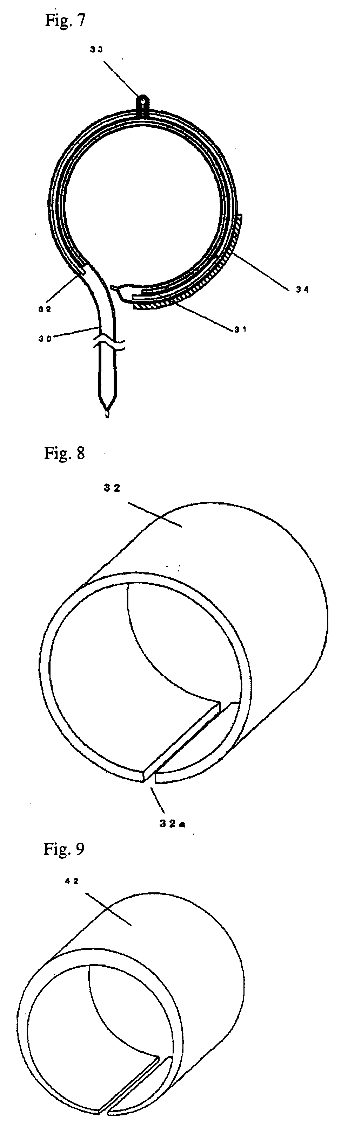

In the curled elastic member 32 shown in Fig. 8, however, there have been the following problems.
1. When the curled elastic member 32 is attached around an arm, the edges of the discontinuous portion 32a of the curled elastic member cut into the flesh of the arm due to its elastic force so that users in particular with thick arm often feel pain.
2. Since the curled elastic member 32 has uniform thickness and the sectional shape thereof is complete round, it cannot deform so as to correspond to a person with thick arm or a person with thin arm and hence is difficult to fit with such arms.

For addressing the above described problems, Japanese Unexamined Patent Publication No. S61-238229 (1986) proposes a curled elastic member 42 as shown in Fig. 9. This curled elastic member 42 is so configured that its thickness gradually increases in a circumferential direction of an arm from the both ends toward the center portion and, and hence the rigidity of the curled elastic member 42 gradually increases. The thickness and rigidity are the maximum at the center portion.

This curled elastic member 42 has somewhat solved the aforementioned problems, however, for an arm whose diameter changes to large extent depending on the part of the arm, or in other words, a largely-inclined arm, such as an upper arm whose diameter is small in the vicinity of the elbow but gradually increases toward the shoulder, the curled elastic member 42 fits with the large diameter part of the arm while leaving a clearance with respect to the small diameter part of the arm. As described above, the curled elastic member 42 cannot sufficiently deform to correspond to the shape of the arm.

The present invention was devised to solve the above-mentioned problems associated with the prior art, and it is an object of the present invention to provide a cuff for a blood pressure monitor with excellent fitting ability which can fit various shapes of arms, inparticular, largely-inclinedarms.

### SUMMARY OF THE INVENTION

In order to achieve the above-mentioned object, a cuff for a blood pressure monitor of the present invention has therein a bladder and a curled elastic member disposed on the outside of the bladder to hold a ring shape of the cuff, and is characterized in that the curled elastic member has nonuniform rigidity in a ring-shaped axial direction of an arm. According to this curled elastic member, since the part having high rigidity is tightly fastened at the time of attachment around an arm and the part having low rigidity conforms to the shape of the arm, it is possible to attach it in conformance with the shape of the arm.

In addition, if the curled elastic member has high rigidity in the part corresponding to the side where the diameter of the arm around which the cuff is to be attached is small, and has low rigidity in the part corresponding to the side where the diameter of the arm is larger, it can be attached in conformance with, in particular, largely-inclined arms.

Furthermore, the curled elastic member may have nonuniform rigidity in a ring-shaped circumferential direction of an arm. As a result of this, the curled elastic member can easily twist and conform to a wide variety of shapes of arms.

Furthermore, the curled elastic member may have low rigidity at an end portion which is to be positioned inside when attached around an arm. As a result of this, the end portion which is brought into contact with an arm at the time of attachment conforms to the shape of the arm, so that pain to the arm can be reduced.

In this curled elastic member, a part having low rigidity may be formed by reducing the thickness. As a result of this, it is possible to readily form regions having different rigidities. Furthermore, in the curled elastic member, a part having low rigidity may be formed by joining at least two members having different shapes and providing a region where at least two members do not overlap with each other. As a result of this, it is possible to deal with different rigidities using more conditions.

Furthermore, in the curled elastic member, a part having low rigidity may be formed by performing punching or boring process on a material. It is possible to set a difference in rigidity more suitably in accordance with the settings such as descending position of hole (bore), density, diameter of hole (or bore) and the like.

### BRIEF DESCRIPTION OF THE DRAWINGS

### [Fig. 1]

A perspective view of a curled elastic member in the first embodiment of the present invention.

### [Fig. 2]

An exploded view of a curled elastic member in the first embodiment of the present invention.

### [Fig. 3]

A perspective view of a curled elastic member in the second embodiment of the present invention.

### [Fig. 4]

An exploded view of a curled elastic member in the second embodiment of the present invention.

### [Fig. 5]

An exploded view of a curled elastic member in the third embodiment of the present invention.

### [Fig. 6]

A perspective view showing appearance of a curled elastic member of a general cuff for a blood pressure monitor.

### [Fig. 7]

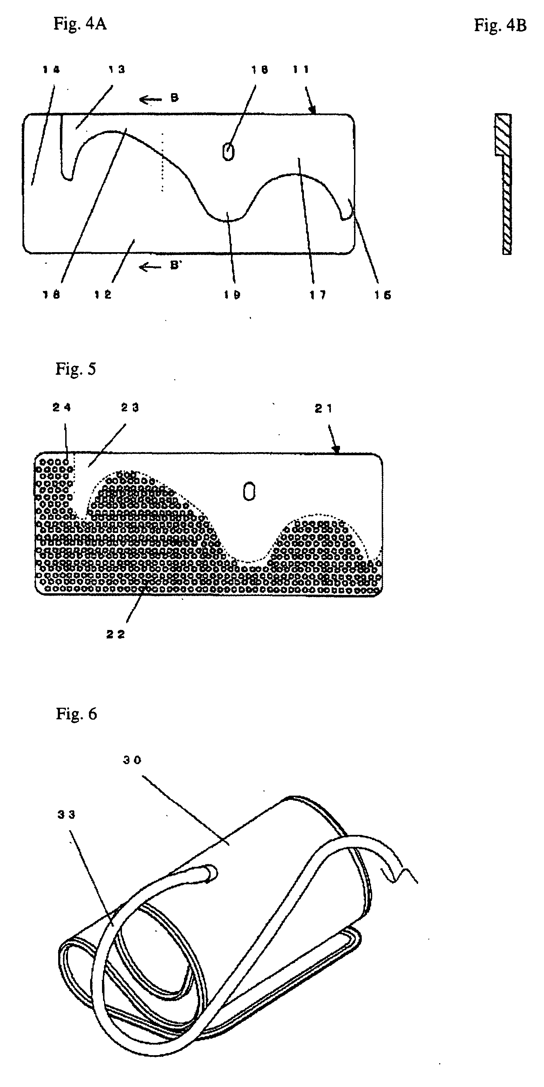

A section view showing the interior of the cuff shown in Fig. 6.

### [Fig. 8]

A perspective view showing a conventional curled elastic member.

### [Fig. 9]

A perspective view showing a conventional curled elastic member.

### DESCRIPTION OF THE PREFERRED EMCODIMENTS

Preferred embodiments of the present invention will be described with reference to the drawings hereinafter. It is to be noted that the cuff for a blood pressure monitor in the present invention has its feature in a curled elastic member thereof, and a structure of the cuff except for the curled elastic member may be a conventional one as shown in Fig. 6. Accordingly, the following explanation focuses on the curled elastic member. The operation associated with this curled elastic member is also maintained in a cuff having the curled elastic member.

### First embodiment

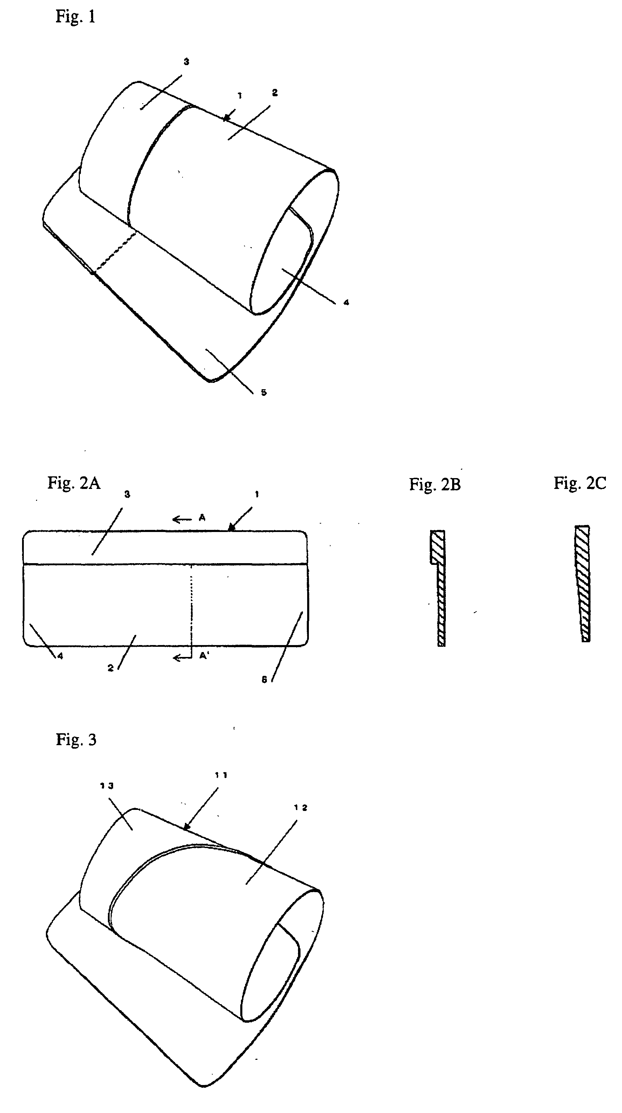

Fig. 1 and Fig. 2A are a perspective view and an exploded view showing a curled elastic member provided for a cuff for a blood pressure monitor, according to the first embodiment of the present invention. A section view viewed from the right direction along the line A-A' of Fig. 2A is shown in Fig. 2B. A curled elastic member 1 is formed of a resin such as PP (polypropylene), and includes two regions having different thicknesses: a thin portion 2 and a thick portion 3. When the curled elastic member 1 is attached around an arm, it is wounded so that one end portion 4 is placed inside another end portion 5. Attention is now directed to an axial direction of an arm in the condition that the curled elastic member 1 is attached around an arm, and it can be seen that one end of the cylindrically wound curled elastic member 1 forms the thin portion 2 and the other end forms the thick portion 3. The thick portion 3 has high rigidity so that it can firmly wind the arm, whereas the thin portion 2 has low rigidity so that it can easily deform in conformance with the shape of the arm in contrast to the thick portion 3. Consequently, when the curled elastic member 1 is oriented so that the thick portion 3 corresponds to the arm part of small diameter and the thin portion 2 corresponds to the arm part of large diameter, and the curled elastic member 1 is wound so that no clearance is left between the thick portion 3 and the arm part of small diameter, the thin portion 2 deforms to closely attach to the arm part of large diameter, resulting that it can be attached around the entire circumference of the arm without leaving any clearance.

In the first embodiment, the rigidity of the curled elastic member 1 is varied in two steps, however, the rigidity may be set more finely, or may be continuously varied by, for example, gradually changing the thickness in the axial direction of an arm from the one end side to the other end side as illustrated in the section view of Fig. 2C.

### Second embodiment

Fig. 3 and Fig. 4A are a perspective view and an exploded view showing a curled elastic member provided for a cuff for a blood pressure monitor, according to the second embodiment of the present invention. A section view viewed from the right direction along the line B-B' of Fig. 4A is shown in Fig. 4B. A curled elastic member 11 includes two regions having different thicknesses: a thin portion 12 and a thick portion 13. When the curled elastic member 11 is attached around an upper arm, it is wounded so that the thin portion 12 is located on the shoulder side and one end portion 14 is placed inside another end portion 15. Attention is now directed to an axial direction of an arm in the condition that the curled elastic member 11 is attached around the upper arm, and then it can be seen that the part corresponding to the shoulder side forms the thin portion 12 and the part corresponding to the elbow side forms the thick portion 13. Accordingly, likewise the curled elastic member 1 according to the first embodiment of the present invention provided for the cuff of a blood pressure monitor, when the thick portion 13 having high rigidity is wound around the arm part near the elbow where the diameter is smaller than that of the part on the shoulder side so as not to leave a clearance, the thin portion 12 having lower rigidity deforms to be closely attached to the arm part on the shoulder side where the diameter is larger than that of the elbow side. As a result of this, it can be attached around a largely-inclined arm without leaving any clearance.

Next, attention is directed to a circumferential direction of an arm, and then it can be seen that on either side of a hole 16 through which a tube connected to a bladder (not shown) is to be inserted into the curled elastic member 11, is provided a narrow portion 17, 18 where the width of the thick portion 13 along the axial direction of an arm is partially narrowed. These narrow portions 17 and 18 can conform with arms having various inclination angles and enables attachment while ensuring higher fitting ability since the rigidity along the axial direction of an arm of the curled elastic member 11 is low in these narrow portions 17 and 18 and hence the curled elastic member 11 can easily twist.

Furthermore, in the vicinity of the hole 16 is formed a wide portion 19 where the width of the thick portion 13 along the axial direction of an arm is elongated, whereby the rigidity is set higher so as to reduce the deformation during pressurizing to the arm by the bladder. Furthermore, as for one end portion 14 and the other end portion 15, the one end portion 14 and its vicinity is formed as the thin portion 12 and the other end 15 and its vicinity is formed as the thick portion 13. When the cured elastic member is wound around an upper arm, the one end portion 14 which is designed to lie inside and come into contact with the arm will conform with the shape of the arm to reduce the pain to the arm since it has low rigidity, while the other end potion 15 will ensure close attachment to the arm since it has high rigidity.

In order to form a curled elastic member having nonuniform rigidity, for example, the thin portion 12 and the thick portion 13 may be integrally formed to make the curled elastic member 11 or the part corresponding to the thin portion 12 may be formed by cutting from a member having the entire shape of the curled elastic member 11 and having the thickness of the thick portion 13. Also, a member having a shape of the part corresponding to the thick portion 13 may be bonded on a member having the entire shape of the curled elastic member 11 formed in a thickness of the thin portion 12. At this time, it is effective to use materials of different rigidities such that the member having the entire shape is formed of a material of low rigidity and the member having a shape of the part corresponding to the thick portion 13 is formed of a material of high rigidity, for example.

The boundary pattern between the thin portion 12 and the thick portion 13 shown in Fig. 4 which is an exploded view is not limited to the illustrated one.

### Third embodiment

Fig. 5 shows an exploded view showing a curled elastic member 21 provided for a cuff for a blood pressure monitor, according to the third embodiment of the present invention . This curled elastic member 21 is another embodiment for implementing the same embodiment as the curled elastic member 11 according to the second embodiment, wherein the rigidities along the axial and circumferential directions of an arm are changed by effecting apunching (boring) process on thematerial. To be more specific, a part 22 which is intended to have low rigidity corresponding to the thin portion 12 in the curled elastic member 11 of the second embodiment is subjected to a punching (boring) process to provide a plurality of holes (or bores) 24, while a part 23 which is intended to have high rigidity corresponding to the thick part 13 is subjected to neither punching nor boring process. As a result of this, the entire thickness of the curled elastic member 21 is uniform, while the rigidity is not uniform in the axial and circumferential directions of an arm and desired rigidity distribution allowing deformation for conforming to the shape of the arm is possible. Accordingly, likewise the curled elastic member 11 according to the second embodiment, the curled elastic member 21 can be closely attached around any arms having a variety of shapes and degrees of inclination.

Operations and effects of the present invention can be achieved more effectively by allowing the position where punching or boring is effected, the processing density, the diameter of bore or hole to be arbitrarily set. The size of bore or hole is not necessarily the same with each other, but large sized and small sized bores or holes may exist in combination. Although either punching or boring process is effected in Fig. 5, processes for making holes and bores may be effected in combination. Accordingly, the pattern of punching or boring process is not limited to the one shown in Fig. 5 as is the same with the second embodiment.

As described above, according to the cuff for a blood pressure monitor of the present invention, it is possible to fittingly attach the cuff around various shapes of arms, in particular largely-inclined arms.

## Claims

1. A cuff for a blood pressure moritor, comprising therein a bladder and a curled elastic member disposed on the outside of the bladder to hold a ring shape of the cuff,
wherein said curled elastic member has nonuniform rigidity in a ring-shaped axial direction of an arm.

2. The cuff for a blood pressure monitor according to claim 1, wherein said curled elastic member has high rigidity in the part corresponding to the side where the diameter of the arm around which the cuff is to be attached is small, and has low rigidity in the part corresponding to the side where the diameter of the arm is larger.

3. The cuff for a blood pressure monitor according to claim 1 or 2 , wherein said curled elastic member has nonuniform rigidity also in a ring-shaped circumferential direction of an arm.

4. The cuff for a blood pressure monitor according to claim 3, wherein said curled elastic member has low rigidity at an end portion which is to be positioned inside when attached around an arm.

5. The cuff for a blood pressure monitor according to any one of claims 1 to 3, wherein a part having low rigidity in said curled elastic member is formed by reducing the thickness.

6. The cuff for a blood pressure monitor according to any one of claims 1 to 3, wherein a part having low rigidity in said curled elastic member is formed by joining at least two members having different shapes and providing a region where said at least two members do not overlap with each other.

7. The cuff for a blood pressure monitor according to any one of claims 1 to 3, wherein a part having low rigidity in said curled elastic member is formed by conducting a punching or boring process on a material.

## Patentansprüche

1. Manschette für ein Blutdruckmessgerät, welche eine Blase und ein geringeltes elastisches Teil enthält, welches auf der Außenseite der Blase angeordnet ist, um eine Ringform der Manschette zu halten,
wobei das geringelte elastische Teil ungleichförmige Steifigkeit in einer ringförmigen axialen Richtung eines Arms hat.

2. Manschette für ein Blutdruckmessgerät nach Anspruch 1, wobei das geringelte elastische Teil hohe Steifigkeit hat in dem Teil, der der Seite entspricht, wo der Durchmesser des Arms, um den die Manschette anzubringen ist, klein ist, und niedrige Steifigkeit in dem Teil, der der Seite entspricht, wo der Durchmesser des Arms größer ist.

3. Manschette für ein Blutdruckmessgerät nach Anspruch 1 oder 2, wobei das geringelte elastische Teil ungleichförmige Steifigkeit auch in einer ringförmigen Umfangsrichtung eines Arms hat.

4. Manschette für ein Blutdruckmessgerät nach Anspruch 3, wobei das geringelte elastische Teil niedrige Steifigkeit an einem Endabschnitt hat, der bei Anbringung um einen Arm innen anzuordnen ist.

5. Manschette für ein Blutdruckmessgerät nach einem der Ansprüche 1 bis 3, wobei ein Teil mit niedriger Steifigkeit in dem geringelten elastischen Teil durch Verminderung der Dicke ausgebildet ist.

6. Manschette für ein Blutdruckmessgerät nach einem der Ansprüche 1 bis 3, wobei ein Teil mit niedriger Steifigkeit in dem geringelten elastischen Teil durch Zusammenfügen von wenigstens zwei Teilen ausgebildet ist, die unterschiedliche Formen haben und einen Bereich liefern, wo die wenigstens zwei Teile einander nicht überlappen.

7. Manschette für ein Blutdruckmessgerät nach einem der Ansprüche 1 bis 3, wobei ein Teil mit niedriger Steifigkeit in dem geringelten elastischen Teil durch Durchführen eines Stanzens oder Bohrens auf einem Material ausgebildet ist.

## Revendications

1. Brassard pour un sphygmomanomètre, comprenant une vessie et un élément élastique bouclé disposé sur l'extérieur de la vessie pour maintenir une forme annulaire du brassard,
dans lequel ledit élément élastique bouclé a une rigidité non uniforme dans une direction axiale annulaire d'un bras.

2. Brassard pour un sphygmomanomètre selon la revendication 1, dans lequel ledit élément élastique bouclé a une haute rigidité dans la partie correspondant au côté où le diamètre du bras autour duquel le brassard doit être fixé est petit, et a une faible rigidité dans la partie correspondant au côté où le diamètre du bras est plus important.

3. Brassard pour un sphygmomanomètre selon la revendication 1 ou 2, dans lequel ledit élément élastique bouclé a une rigidité non uniforme aussi dans une direction circonférentielle annulaire d'un bras.

4. Brassard pour un sphygmomanomètre selon la revendication 3, dans lequel ledit élément élastique bouclé a une faible rigidité sur une partie d'extrémité qui doit être positionnée à l'intérieur lorsqu'il est fixé autour d'un bras.

5. Brassard pour un sphygmomanomètre selon l'une quelconque des revendications 1 à 3, dans lequel une partie ayant une faible rigidité dans ledit élément élastique bouclé est formée en réduisant l'épaisseur.

6. Brassard pour un sphygmomanomètre selon l'une quelconque des revendications 1 à 3, dans lequel une partie ayant une faible rigidité dans ledit élément élastique bouclé est formée en joignant au moins deux éléments ayant différentes formes et en fournissant une région où lesdits au moins deux éléments ne se chevauchent pas.

7. Brassard pour un sphygmomanomètre selon l'une quelconque des revendications 1 à 3, dans lequel une partie ayant une faible rigidité dans ledit élément élastique est formée en effectuant un procédé de perforation ou d'alésage sur un matériau.
